(19) Europäisches Patentamt

European Patent Office

Office européen des brevets

(11) **EP 1 023 032 B1**

(12) **EUROPEAN PATENT SPECIFICATION**

(45) Date of publication and mention
of the grant of the patent:
**02.01.2002   Bulletin 2002/01**

(51) Int Cl.$^7$: **A61K 6/033**, A61L 24/00,
A61L 27/00

(21) Application number: **98954344.2**

(22) Date of filing: **06.10.1998**

(86) International application number:
**PCT/EP98/06330**

(87) International publication number:
**WO 99/17710 (15.04.1999 Gazette 1999/15)**

(54) **HYDRAULIC SURGICAL CEMENT**

HYDRAULISCHER CHIRURGISCHER ZEMENT

CIMENT CHIRURGICAL HYDRAULIQUE

(84) Designated Contracting States:
**AT BE CH DE DK ES FI FR GB IE IT LI LU MC NL
PT SE**

(30) Priority:  **07.10.1997   WOPCT/EP97/05495**

(43) Date of publication of application:
**02.08.2000   Bulletin 2000/31**

(73) Proprietors:
• **Dr.h.c. Robert Mathys Stiftung
2544 Bettlach (CH)**
• **Stratec Medical AG
4436 Oberdorf (CH)**

(72) Inventors:
• **LEMAITRE, Jaques
1012 Lausanne (CH)**
• **BOHNER, Marc
8051 Zürich (CH)**

• **VAN LANDUYT, Pascale
1024 Ecublens (CH)**

(74) Representative: **Lusuardi, Werther Giovanni, Dr.
Dr. Lusuardi AG, Kreuzbühlstrasse 8
8008 Zürich (CH)**

(56) References cited:
**EP-A- 0 543 765          DE-A- 4 216 496
GB-A- 2 260 977          US-A- 5 152 836**

• **MIRTCHI A A ET AL: "CALCIUM PHOSPHATE
CEMENTS: ACTION OF SETTING REGULATORS
ON THE PROPERTIES OF THE -TRICALCIUM
PHOSPHATE- MONOCALCIUM PHOSPHATE
CEMENTS" BIOMATERIALS, vol. 10, no. 9, 1
November 1989, pages 634-638, XP000081742
cited in the application**

**Description**

This invention concerns a cement for surgical purposes in accordance with the pre-characterising portion of Claim 1, a method for producing brushite as temporary bone replacement material according to the pre-characterising portion of Claim 34 and the temporary bone replacement material obtained by said method according to Claim 35.

A number of such hydraulic cements based on calcium phosphates for use in surgery are known in the prior art; they are prepared from two components (powder/liquid) by mixing them intra-operatively and applying them in pasteous consistency to the appropriate site where they harden in situ. The disadvantages of the prior art hydraulic cements based calcium phosphates are:

impracticable short setting times which do not allow their use for elaborate surgical procedures;
poor injectability, i.e. the fresh cement paste tends to clog the injection needle, and/or disintegrates in contact with physiological liquids, which prevents its implantation by minimal invasive surgery procedures;
low compacity, i.e. current hydraulic cements need larger amounts of mixing water in order to have them injectable or to confer them a convenient setting time, which results in very low ultimate mechanical strength after hardening.

In the US-A-4 880 610 CONSTANTZ a method is disclosed for making an in situ calcium phosphate mineral composition by combining water-free phosphoric acid crystals with a calcium source which leads to a hydroxyapatite. It is clear that the use of 100% phosphoric acid in the operating room and the application of a paste containing 100% phosphoric acid in the human body must be considered a not ideal procedure which requires improvement. Furthermore the hydroxyapatite material produced by this known method will have a long resorption period which is not commensurate to the rate of the bone remodelling. The disadvantage of prolonged resorption is that the bone treated by cement will remain for a prolonged time in abnormal biomechanical situation, which may develop secondary post-operational problems. Furthermore the unresorbed cement may still break down in pieces or fragments after prolonged mechanical loading, which increases the probability of post-operational complications, e.g. aseptic inflammatory reactions. The resorption rate of the ideal cement should match as closely possible the spontaneous rate of new bone formation, which is around 20 micrometers per day.

From GB-2 260 977 MITSUBISHI a calcium phosphate composition is known using alpha-TCP particles. Alpha-TCP particles are much more reactive than beta-TCP particles and therefore lead to a setting time - when admixed to monocalcium phosphate monohydrate and water - that is much too fast (a few seconds), and hence difficult to control.

From an article of MIRCHI A A ET AL. appeared in Biomaterial 1989, Vol. 10, No. 9, 1 November 1989, pages 634 - 638, a calcium phosphate cement is known with commercially available MCPM and β-TCP particles the Ca/P ratio of which is 1,50.
The disadvantages of β-TCP particles with a Ca/P ratio of 1,50 is their relatively high reactivity which makes them inappropriate for a surgical cement.

The invention as claimed aims at solving the above described problems. The present invention provides a cement for surgical purposes as defined in Claim 1, a method for producing a temporary bone replacement material as defined in Claim 34 and a temporary bone replacement material as defined in claim 35.

The various features of novelty which characterize the invention are pointed out with particularity in the claims annexed to and forming part of this disclosure. For the better understanding of the invention, its operating advantages and specific objects attained by its use, reference should be done to the accompanying examples in which preferred embodiments of the invention are illustrated in detail.

The first component of the cement according to the invention comprises besides beta tertiary calciumphosphate $\beta\text{-}Ca_3(PO_4)_2$ (β-TCP) particles one of the following substances:

a) monocalcium phosphate $Ca(H_2PO_4)_2$ (MCPA) particles; or
b) monocalcium phosphate monohydrate $Ca(H_2PO_4)_2 \cdot H_2O$ (MCPM) particles;
c) or phosphoric acid.

Alternative a) and b) are the preferred ones; the phosphoric acid may be used either in solid or in liquid form.

The Ca/P atomic ratio of the β-TCP particles of the first component is preferably comprised between 1,35 to 1,499. Purposefully it is comprised between 1,45 to 1,47 and typically between 1,455 to 1,465. The advantage of the Ca/P atomic ratio used in the invention is the lower reactivity of the β-TCP particles. A lower Ca/P atomic ratio - below 1,5 - can be achieved in avarious ways. One possiblity is the calcination and sintering of DCP $[CaHPO_4]$/hydroxyapatite $[Ca_5(PO_4)_3OH]$) mixtures. The Ca/P ratio is then controlled by teh proportion of the two components. To obtain a Ca/P ratio of exactly 1,50 one should mix 10 g of DCP with 36,91 g HA. To obtain a Ca/P ratio of 1,35 one has to mix 10 g DCP with 13,6 g of HA. Alternatively DCP could be replaced by DCPD $(CaHPO_4 \cdot H_2O)$, MCPM $[Ca(H_2PO_4)_2 \cdot H_2O]$,

CaPP ($Ca_2P_2O_7$) or OCP [$Ca_8H_2PO_4)_6 \cdot 5H_2O$]. The beta-TCP particles with a Ca/P ration inferior to 1,50 can also be obtained by adding small amounts of DCP, DCPD, MCPM or OCP to a pure beta-TCP powder, and then mix and sinter the mixture to homogenize it.

Onother way is to add minor amounts of $Na_4P_2O_7 \cdot 10\ H_2O$ (NaPPH) to the β-TCP particles which retard their dissolution in a synergetic way. Consequently the setting time of the hydraulic cement is significantly increased. The presence of minor amounts of CaPPH increases also the sintering ability of the β-TCP particles, hence enabling the production of dense β-TCP particles. Non-dense β-TCP particles absorb the mixing liquid during setting. As a following, more mixing liquid must be used to knead the cement, provoking a decrease of the mechanical properties of the cement after hardening.

If the amount of CaPPH is too large (Ca/P ratio below 1,35) the mechanical properties of the cement decrease drastically.

The mean specific surface area of the β-TCP particles must be less than 10 $m^2$/g otherwise the cement has poor mechanical properties - due to a large porosity resulting from the large volume of mixing liquid - and a setting time which is too short for practical purposes.

Setting time of the cement according to the invention as measured at 25 C° should be at least 2 minutes, typically at least 3 minutes and preferably at least 5 minutes.

According to a preferred embodiment of the invention the first component is divided into two subcomponents A and B, subcomponent A comprising the MCPA and/or MCPM particles and subcomponent B comprising the β-TCP particles and the setting rate controller.

The second component comprises water and may further comprise orthophosphoric acid (OPA) and/or sulphuric acid (SA), which again take the function of a setting rate controller and also lead to an improved microstructure of the final brushite (DCPD) crystals.

After mixing the three components a hardened mass is formed comprising brushite $CaHPO_4 \cdot 2\ H_2O$ (DCPD), which based on its solubility is used to accelerate the resorption rate compared to HA.

The total weight $W_{TCP}$ of the β-TCP particles of the first and third components should preferably be larger than the stoichiometric weight

$$W_T = W_{MCPA}/0,7546 + W_{MCPM}/0,8127 + W_{OPA}/0,3159 + W_{SA}/0.3162$$

where $W_{MCPA}$, $W_{MCPM}$, $W_{OPA}$ and $W_{SA}$ are, respectively, the weights of MCPA, MCPM, OPA and SA used.

Further the weight $W_{TCP}$ should be in the range of $1,2\ W_T \leq W_{TCP} \leq 2,0\ W_T$.

The first component may further comprise a setting rate controller chosen from the group of sodium pyrophosphate, potassium pyrophosphate, sodium acetate, potassium acetate, sodium citrate, potassium citrate, sodium phosphocitrate, potassium phosphocitrate, sodium sulphate or potassium sulphate, calcium sulphate hemihydrate $CaSO_4 \cdot 0,5\ H_2O$ (CSH), sodium pyrophosphate $Na_4P_2O_7 \cdot 10\ H_2O$ (NaPPH), sodium dihydrogen pyrophosphate $Na_2H_2P_2O_7$ (NaHPP), calcium pyrophosphate $Ca_4P_2O_7$ (CaPP), magnesium sulphate and sodium or potassium biphosphonate.

In a further preferred embodiment of the invention a third component consisting of particles having an average diameter which is larger than the average diameter of said beta tertiary calciumphosphate β-$Ca_3(PO_4)_2$ (β-TCP) particles of said first component is added. This leads to conglomerate structure of the finally set cement, whereby the third component particles are embedded in the brushite matrix formed by the setting process. The average particle diameter of said third component should be at least two times larger, preferably at least 10 times larger compared to the average diameter of the beta tertiary calciumphosphate β-$Ca_3(PO_4)_2$ (β-TCP) particles of the first component. Preferably the average particle diameter of said third component should be in the range of 50 to 2000 μm. The particles of the third component may consist of hydroxyapatite particles or of polymeric particles, e.g. lactides, polysaccharides, collagenes or proteins.

In a further preferred embodiment of the invention two different types of β-TCP particles are used, the first type being particles having a median particle size of 5 μm with less than 10 volume % of the particles being smaller than 1 μm; and the second type being particles having an average diameter in the range of 150 to 500 μm, preferably in the range of 250 to 400 μm.

The average particle diameter of said third component should be in the range of 50 to 2000 μm, preferably between 250 and 750 μm.

The volume $V_L$ of the second component should preferably be equal or superior than the volume $V_T = (W_{MCPA}$ x 0,615 + $W_{MCPM}$ x 0,5 + $W_{OPA}$ x 1,102 + $W_{SA}$ x 1,101) ml/g of the first component. The volume $V_L$ is typically in the range of $0,5\ V_T \leq V_L \leq 10,0\ V_T$, preferably in the range of $1,2\ V_T \leq V_L \leq 2,0\ V_T$.

One of the two components may further comprise a biodegradable polymer for controlling the consistency of the cement paste resulting from mixing of the two components, and its cohesion in physiological liquids.

The biodegradable polymer may be selected from the group of polysaccharide derivatives, preferably hyaluronic acid, dextran, hydroxypropyl-methyl cellulose; chitin derivatives, preferably chitosan; xanthan gum; agarose; polyethyleneglycols (PEG), polyhydroxyethylenemethacrylats (HEMA), synthetic and natural proteins or collagens.

The first component may further comprise pharmaceutically or physiologically active substances, preferably selected from the group of antibiotics, anti-inflammatory, anti-cancer drugs and bone growth factors. The antibiotics is preferably a gentamycin or a gentamycin salt, typically gentamycin sulphate. Other gentamycin salts can be used provided their solubility is in the range of 100 to 2500 mg/l.

The antibiotics is selected from the group of aminoglycosides, vancomycins, gentamycins or salts thereof, preferably gentamycin sulphate or gentamycin crobefat.

The cements according to the invention may be used as bone substitute in dental and maxillofacial surgery (alveolar ridge reconstruction, dental socket filling), for orthopaedic applications (bone fracture repair, bone augmentation) and for local drug delivery (antibiotics, anti-inflammatory and anti-cancer drugs).

In a preferred embodiment the particles of the third component are made from another material than beta tertiary calciumphosphate $\beta$-$Ca_3(PO_4)_2$ ($\beta$-TCP).

The particles of said third component are preferably made from a material selected from the group of: hydroxylapatite; biphasic calcium phosphonate (BCP) (HA/$\beta$-TCP) mixtures; bioglasses; or polymeric materials.

The advantage is the differential degradation of such a cement. The matrix of the cement is degraded faster than the residual granulates. This is particularly useful for the application in the osteoporose field or for the ridge reconstruction of the jaw, where a slower degrading granulate, e.g.made from hydroxyapatite or BCP is desired.

Five specific examples are reported below for producing the temporary bone replacement materials according to the invention.

Example 1

The first component (containing the powdered particles) consists of two separate subcomponents A and B.

Subcomponent A consist of:

0,80 g of monocalcium phosphate monohydrate $Ca(H_2PO_4)_2 \cdot H_2O$ MCPM particles.

Subcomponent B is a mixture of:

1,20 g of beta tertiary calciumphosphate $\beta$-$Ca_3(PO_4)_2$ ($\beta$-TCP) particles having a Ca/P atomic ratio in the range of 1,44 to 1,47. The median particle size of the $\beta$-TCP particles is 5 $\mu$m and its specific surface area is less than 2 $m^2$/g; and

0,012 g sodium pyrophosphate $Na_2H_2P_2O_7$.

The second component (containing the liquid) consists of 0,80 to 0,90 ml of a 0,10 M aqueous sulphuric acid solution.

The third component - which may be admixed to the first component as Subcomponent C - contains 0,5 g ($\beta$-TCP) particles with a diameter comprised in the range of 0,5 to 0,71 mm and a Ca/P atomic ratio in the range of 1,46. The powdered subcomponents are sterilized by gamma-irradiation. The liquid component is prepared in sterile conditions with sterile materials.

The hydraulic cement paste is prepared by using a pestle. Subcomponent A is carefully mixed with the liquid component in a mortar for approximately 1 minute. Thereafter subcomponent B is added and kneaded carefully with a spatula for about a minute, until a uniform paste is obtained.

The paste can be used for about 10 minutes, depending on ambient temperature; the higher the temperature, the shorter the setting time. Using refrigerated material and preparation instruments helps in prolonging the available working time.

Example 2

The first component (containing the powdered particles) consists of 1,3 g of $\beta$-TCP particles with an average diameter of 6 $\mu$m and a Ca/P molar ratio of 1,41 and 0,7 g of MCPM. The third component is added to the first component and comprises 0,3 g of $\beta$-TCP particles with an average diameter comprised in the range of 0,35 to 0,50 mm and a Ca/P molar ratio of 1,42. The second (liquid) component is a solution of 100 mg gentamycin sulphate in 1,6 ml of water.

The powder/liquid components were mixed together for 30 seconds and the resulting cement paste was filled into a syringe for application to a bone defect.

Example 3

The first component (containing the powdered particles) consists of 1,3 g of β-TCP particles with an average diameter of 6 μm and a Ca/P molar ratio of 1,41 and 0,7 g of MCPM. The third component is added to the first component and comprises 0,8 g of hydroxyapatite (HA) particles with an average diameter comprised in the range of 0,35 to 0,50 mm. The second (liquid) component is a solution of 100 mg gentamycin sulphate in 1,6 ml of a 0,16 M aqueous sulphuric acid solution.
The powder/liquid components were mixed together for 30 seconds and the resulting cement paste was filled into a syringe for application to a bone defect.

Example 4

1,5 g of β-TCP particles with an average diameter of 2 μm and a Ca/P molar ratio of 1,46, 0,3 g β-TCP particles with an average diameter comprised in the range of 0,35 to 0,50 mm and a Ca/P molar ratio of 1,42 and 1,5 ml of a solution of $H_3PO_4$ 3M, $H_2SO_4$ 0,1 M and $Na_2H_2P_2O_7$ 0,1 M were mixed together for 30 seconds, placed into a syringe and injected into a bony cavity.

Example 5

1,5 g of β-TCP particles with an average diameter of 2 μm and a Ca/P molar ratio of 1,46, 0,3 g hydroxyapatite (HA) particles with an average diameter comprised in the range of 0,35 to 0,50 mm and 1,5 ml of a solution of $H_3PO_4$ 3M, $H_2SO_4$ 0,1 M and $Na_2H_2P_2O_7$ 0,1 M were mixed together for 30 seconds, placed into a syringe and injected into a bony cavity.

**Claims**

1. Cement for surgical purposes comprising

   a first component comprising

   beta tertiary calciumphosphate $\beta-Ca_3(PO_4)_2$ (β-TCP) particles; and

   monocalcium phosphate $Ca(H_2PO_4)_2$ (MCPA) or monocalcium phosphate monohydrate $Ca(H_2PO_4)_2 \cdot H_2O$ (MCPM) particles or phosphoric acid;

   a second component comprising
     water; and

   a third component comprising particles having an average diameter which is larger than the average diameter of said beta tertiary calciumphosphate $\beta-Ca_3(PO_4)_2$ (β-TCP) particles of said first component,

   whereby upon mixing of said three components a hardened mass comprising brushite $CaHPO_4 \cdot 2H_2O$ (DCPD) is formed,

   **characterized in that**

   a) the β-TCP particles have a mean specific surface area of less than 10,000 $m^2/g$;

   b) the third component is a calcium phosphate with a Ca/P ratio different from 1,5;

   c) the third component constitutes 1 to 99 volume-percent of said hardened mass; and

   d) the first component further comprises a setting rate controller.

2. Cement according to claim 1, **characterized in that** said first component comprises beta tertiary calciumphosphate $\beta-Ca_3(PO_4)_2$ (β-TCP) particles and monocalcium phosphate $Ca(H_2PO_4)_2$ (MCPA) or monocalcium phosphate monohydrate $Ca(H_2PO_4)_2 \cdot H_2O$ (MCPM) particles.

3. Cement according to claim 1 or 2, **characterized in that** the setting time of said cement as measured at 25 C° is at least 2 minutes.

4. Cement according to claim 3, **characterized in that** the setting time of said cement as measured at 25 C° is at least 3 minutes.

5. Cement according to claim 4, **characterized in that** the setting time of said cement as measured at 25 C° is at least 5 minutes.

6. Cement according to one of the claims 1 to 5, **characterized in that** the $\beta$-TCP particles have a mean specific surface area of 0,0137 - 2,000 m$^2$/g.

7. Cement according to claim 6, **characterized in that** the $\beta$-TCP particles have a mean specific surface area of 0,8 - 1,5 m2/g.

8. Cement according to claim 7, **characterized in that** the first component is divided into three subcomponents X, Y and Z, subcomponent X comprising the MCPA and/or MCPM particles, subcomponent Y comprising the $\beta$-TCP particles and the setting rate controller and subcomponent Z comprising the third component.

9. Cement according to claim 7 or 8, **characterized in that** the setting rate controller is chosen from the group of sodium pyrophosphate, potassium pyrophosphate, sodium acetate, potassium acetate, sodium citrate, potassium citrate, sodium phosphocitrate, potassium phosphocitrate, sodium sulphate or potassium sulphate, calcium sulphate hemihydrate $CaSO_4 \cdot 0,5 \, H_2O$ (CSH), sodium pyrophosphate $Na_4P_2O_7 \cdot 10 \, H_2O$ (NaPPH), sodium dihydrogen pyrophosphate $Na_2H_2P_2O_7$ (NaHPP), calcium pyrophosphate $Ca_4P_2O_7$ (CaPP), magnesium sulphate and sodium or potassium biphosphonate.

10. Cement according to one of the claims 1 to 9, **characterized in that** said second component further comprises orthophosphoric acid (OPA) and/or sulphuric acid (SA).

11. Cement according to one of the claims 1 to 10, **characterized in that** the total weight $W_{TCP}$ of the $\beta$-TCP particles of the first and third component is larger than the weight

$$W_T = W_{MCPA}/0,7546 + W_{MCPM}/0,8127 + W_{OPA}/0,3159 + W_{SA}/0,3162$$

where $W_{MCPA}$, $W_{MCPM}$, $W_{OPA}$ and $W_{SA}$ are, respectively, the weights of MCPA, MCPM, OPA and SA used.

12. Cement according to claim 11, **characterized in that** the weight $W_{TCP}$ is in the range of $1,2 \, W_T \leq W_{TCP} \leq 10,0 \, W_T$.

13. Cement according to claim 12, **characterized in that** the weight $W_{TCP}$ is in the range of $2 \, W_T \leq W_{TCP} \leq 5 \, W_T$.

14. Cement according to one of the claims 1 to 13, **characterized in that** the average particle diameter of said third component is at least two times, preferably at least 10 times larger than the average diameter of said beta tertiary calciumphosphate $\beta$-$Ca_3(PO_4)_2$ ($\beta$-TCP) particles of said first component.

15. Cement according to one of the claims 1 to 14, **characterized in that** the average particle diameter of said third component is in the range of 50 to 2000 $\mu$m, preferably between 250 and 750 $\mu$m.

16. Cement according to one of the claims 1 to 15, **characterized in that** said third component comprises hydroxyapatite particles.

17. Cement according to one of the claims 1 to 15, **characterized in that** said third component comprises polymeric particles.

18. Cement according to one of the claims 1 to 17, **characterized in that** two different types of $\beta$-TCP particles are used,

the first type being particles having a median particle size of 5 $\mu$m with less than 10 volume % of the particles being smaller than 1 µm; and

the second type being particles having an average diameter in the range of 150 to 500 µm, preferably in the range of 250 to 400 µm.

**19.** Cement according to one of the claims 1 to 18, **characterized in that** the Ca/P atomic ratio of the β-TCP particles of said first component is comprised between 1,350 to 1,499, preferably between 1,45 to 1,47.

**20.** Cement according to claim 19, **characterized in that** said Ca/P atomic ratio is comprised between 1,455 to 1,465.

**21.** Cement according to one of the claims 1 to 20, **characterized in that** one of the two components further comprises a biodegradable polymer.

**22.** Cement according to claim 21, **characterized in that** the biodegradable polymer is selected from the group of

polysaccharide derivatives, preferably hyaluronic acid, dextran, hydroxypropyl-methyl cellulose;
chitin derivatives, preferably chitosan;
xanthan gum;
agarose;
polyethyleneglycols (PEG), polyhydroxyethylenemethacrylats (HEMA);
synthetic and natural proteins; or
collagens.

**23.** Cement according to one of the claims 1 to 22, **characterized in that** the volume $V_L$ of the second component is equal or larger than the volume

$$V_T = (W_{MCPA} \times 0,615 + W_{MCPM} \times 0,5 + W_{OPA} \times 1,102 + W_{SA} \times 1,101) \text{ ml/g}$$

of the first component.

**24.** Cement according to one of the claims 1 to 23, **characterized in that** the relation of volume $V_L$ of the second component and of $V_T$ of the first component is in the range of $0,5 \, V_T \leq V_L \leq 10,0 \, V_T$, preferably in the range of $1,2 \, V_T \leq V_L \leq 2,0 \, V_T$.

**25.** Cement according to one of the claims 1 to 24, **characterized in that** said first component further comprises pharmaceutically or physiologically active substances, preferably selected from the group of antibiotics, anti-inflammatory, anti-cancer drugs and bone growth factors.

**26.** Cement according to claim 25, **characterized in that** said antibiotics is selected from the group of:
aminoglycosides, vancomycins, gentamycins or salts thereof, preferably gentamycin sulphate or gentamycin crobefat.

**27.** Cement according to claim 26, **characterized in that** said antibiotics is a mixture of aminoglycosides, preferably a mixture of vancomycin with gentamycin.

**28.** Cement according to one of the claims 1 to 27, **characterized in that** the first component comprises at least 50 weight percent of beta tertiary calciumphosphate $\beta\text{-}Ca_3(PO_4)_2$ (β-TCP).

**29.** Cement according to one of the claims 1 to 28, **characterized in that** the particles of said third component are made from another material than beta tertiary calciumphosphate $\beta\text{-}Ca_3(PO_4)_2$ (β-TCP).

**30.** Cement according to claim 29, **characterized in that** the particles of said third component are made from a material selected from the group of:

hydroxyapatite;
biphasic calcium phosphate (HA/β-TCP) mixtures;
bioglasses;
or polymeric materials.

31. Cement according to one of the claims 1 to 30, **characterized in that** the third component constitutes 1 to 99 volume-percent of said hardened mass.

32. Cement according to one of the claims 1 to 31, **characterized in that** the third component is a calcium phosphate with a Ca/P ratio lower than 1,5, preferably in the range of 1,35 to 1,49.

33. Cement according to one of the claims 1 to 31, **characterized in that** the third component is a calcium phosphate with a Ca/P ratio higher than 1,5, preferably hydroxyapatite.

34. Method for producing a matrix of brushite $CaHPO_4 \cdot 2H_2O$ (DCPD) as temporary bone replacement material **characterized in that** said three components according to one of the claims 1 to 33 are mixed together and allowed to harden.

35. Temporary bone replacement material obtained by the method according to claim 34, **characterized in that** it comprises brushite $CaHPO_4 \cdot 2H_2O$ (DCPD).

36. Temporary bone replacement material according to claim 35, **characterized in that** it comprises beta tertiary calciumphosphate $\beta$-$Ca_3(PO_4)_2$ ($\beta$-TCP) particles embedded in said brushite matrix.

**Patentansprüche**

1. Zement für chirurgische Zwecke, bestehend aus

    einer ersten-Komponente, bestehend aus

        beta-tertiärem Calciumphosphatpartikeln $\beta$-$Ca_3$ $(PO_4)_2$ ($\beta$-TCP); und
        Monocalciumphosphat- $Ca(H_2PO_4)_2$ (MCPA) oder
        Monocalciumphosphatmonohydratpartikel $Ca(H_2PO_4)_2 \cdot H_2O$ (MCPM)
        oder Phosphorsäure;

    einer zweiten Komponente, bestehend aus
        Wasser; und
    einer dritten Komponente aus Partikeln mit einem durchschnittlichen Durchmesser, der größer ist als der durchschnittliche Durchmesser der besagten beta-tertiären Calciumphosphatpartikel $\beta$-$Ca_3(PO_4)_2$ ($\beta$-TCP) der besagten ersten Komponente,
    wobei beim Mischen der besagten drei Komponenten eine gehärtete Masse aus Brushit $CaHPO_4 \cdot 2H_2O$ (DCPD) gebildet wird,

    **dadurch gekennzeichnet, daß**

        a) die $\beta$-TCP-Partikel eine durchschnittliche spezifische Oberfläche von weniger als 10,000 $m^2/g$ haben;

        b) die dritte Komponente ein Calciumphosphat mit einem Ca/P-Verhältnis ist, das nicht von 1,5 entspricht;

        c) die dritte Komponente 1 bis 99 Volumenprozent der besagten gehärteten Masse bildet; und

        d) die erste Komponente außerdem einen Erhärtungsratenregulator enthält.

2. Zement nach Anspruch 1, **dadurch gekennzeichnet, daß** die besagte erste Komponente beta-tertiäre Calciumphosphatpartikeln $\beta$-$Ca_3$ $(PO_4)_2$ ($\beta$-TCP) und Monocalciumphosphat- $Ca(H_2PO_4)_2$ (MCPA) oder Monocalciumphosphatmonohydratpartikel $Ca(H_2PO_4)_2 \cdot H_2O$ (MCPM) enthält.

3. Zement nach Anspruch 1 oder 2, **dadurch gekennzeichnet, daß** die Erhärtungszeit des besagten Zements, gemessen bei 25 °C, mindestens 2 Minuten beträgt.

4. Zement nach Anspruch 3, **dadurch gekennzeichnet, daß** die Erhärtungszeit des besagten Zements, gemessen bei 25 °C, mindestens 3 Minuten beträgt.

**5.** Zement nach Anspruch 4, **dadurch gekennzeichnet, daß** die Erhärtungszeit des besagten Zements, gemessen bei 25 °C, mindestens 5 Minuten beträgt.

**6.** Zement nach einem der Ansprüche 1 bis 5, **dadurch gekennzeichnet, daß** die β-TCP-Partikel eine durchschnittliche spezifische Oberfläche von 0,0137 - 2,000 $m^2$/g haben.

**7.** Zement nach Anspruch 6, **dadurch gekennzeichnet, daß** die β-TCP-Partikel eine durchschnittliche spezifische Oberfläche von 0,8 - 1,5 $m^2$/g haben.

**8.** Zement nach Anspruch 7, **dadurch gekennzeichnet, daß** die erste Komponente in drei Subkomponenten X, Y und Z unterteilt ist, wobei die Subkomponente X die MCPA- und/oder MCPM-Partikel, die Subkomponente Y die β-TCP-Partikel und den Erhärtungsratenregulator und die Subkomponente Z die dritte Komponente enthält.

**9.** Zement nach Anspruch 7 oder 8, **dadurch gekennzeichnet, daß** der Erhärtungsratenregulator aus der Gruppe Natriumpyrophosphat, Kaliumpyrophosphat, Natriumacetat, Kaliumacetat, Natriumcitrat, Kaliumcitrat, Natriumphosphocitrat, Kaliumphosphocitrat, Natriumsulphat oder Kaliumsulphat, Calciumsulphathemihydrat $CaSo_4 . 0,5 H_2O$ (CSH), Natriumpyrophosphat $Na_4P_2O_7$. 10 $H_2O$ (NaPPH), Natriumdihydrogenpyrophosphat $Na_2H_2P_2O_7$ (NaHPP), Calciumpyrophosphat $Ca_4P_2O_7$ (CaPP), Magnesiumsulphat und Natrium- oder Kaliumbiphosphonat gewählt wird.

**10.** Zement nach einem der Ansprüche 1 bis 9, **dadurch gekennzeichnet, daß** die besagte zweite Komponente zusätzlich Orthophosphorsäure (OPA) und/oder Schwefelsäure (SA) enthält.

**11.** Zement nach einem der Ansprüche 1 bis 10, **dadurch gekennzeichnet, daß** das Gesamtgewicht $W_{TCP}$ der β-TCP-Partikel der ersten und dritten Komponente über dem Gewicht $W_T = W_{MCPA}/0,7546 + W_{MCPM}/0,8127 + W_{OPA}/0,3159 + W_{SA}/0.3162$ liegt, wobei $W_{MCPA}$, $W_{MCPM}$, $W_{OPA}$ und $W_{SA}$ respektive die angewandten Gewichte von MCPA, MCPM, OPA und SA sind.

**12.** Zement nach Anspruch 11, **dadurch gekennzeichnet, daß** das Gewicht $W_{TCP}$ im Bereich von $1,2 W_T \leq W_{TCP} \leq 10,0 W_T$ liegt.

**13.** Zement nach Anspruch 12, **dadurch gekennzeichnet, daß** das Gewicht $W_{TCP}$ im Bereich von $2 W_T \leq W_{TCP} \leq 5 W_T$ liegt.

**14.** Zement nach einem der Ansprüche 1 bis 13, **dadurch gekennzeichnet, daß** der durchschnittliche Partikeldurchmesser der besagten dritten Komponente mindestens doppelt so groß, und vorzugsweise 10mal größer als der Durchschnittsdurchmesser der beta-teriären Calciumphosphatpartikel $β-Ca_3 (PO_4)_2$ (β-TCP) der besagten ersten Komponente ist.

**15.** Zement nach einem der Ansprüche 1 bis 14, **dadurch gekennzeichnet, daß** der durchschnittliche Partikeldurchmesser der besagten dritten Komponente im Bereich von 50 bis 2000 μm, vorzugsweise zwischen 250 und 750 μm, liegt.

**16.** Zement nach einem der Ansprüche 1 bis 15, **dadurch gekennzeichnet, daß** die besagte dritte Komponente Hydroxyapatitpartikel enthält.

**17.** Zement nach einem der Ansprüche 1 bis 15, **dadurch gekennzeichnet, daß** die besagte dritte Komponente polymerischen Partikel enthält.

**18.** Zement nach einem der Ansprüche 1 bis 17, **dadurch gekennzeichnet, daß** zwei verschiedene Typen von β-TCP-Partikeln verwendet werden,

wobei der erste Partikeltyp eine mittleren Partikelgröße von 5 μm hat, mit weniger als 10 Volumenprozent der Partikel kleiner als 1 μm; und
der zweite Partikeltyp einen mittleren Durchmessers im Bereich von 150 bis 500 μ m aufweist, vorzugsweise im Bereich von 250 bis 400 μm.

**19.** Zement nach einem der Ansprüche 1 bis 18, **dadurch gekennzeichnet, daß** das Ca/P-Atomverhältnis der β-TCP-Partikel der besagten ersten Komponente zwischen 1,350 und 1,499, vorzugsweise zwischen 1,45 und 1,47, liegt.

**20.** Zement nach Anspruch 19, **dadurch gekennzeichnet, daß** das besagte Ca/P-Atomverhältnis zwischen 1,455 und 1,465 liegt.

**21.** Zement nach einem der Ansprüche 1 bis 20, **dadurch gekennzeichnet, daß** eine der beiden Komponenten zudem ein biologisch abbaubares Polymer enthält.

**22.** Zement nach Anspruch 21, **dadurch gekennzeichnet, daß** das biologisch abbaubare Polymer aus der Gruppe Polysaccharidderivate ausgewählt wird, vorzugsweise

   Hyaluronsäure, Dextran, Hydroxypropylmethylcellulose;
   Chitinderivate, vorzugsweise Chitosan;
   Xanthangummi;
   Agarose;
   Polyethylenglykol (PEG), Polyhydroxyethylenemethacrylat (HEMA), synthetische und natürliche Proteine;
   oder Kollagene.

**23.** Zement nach einem der Ansprüche 1 bis 22, **dadurch gekennzeichnet, daß** das Volumen $V_L$ der zweiten Komponente gleich oder größer ist als das Volumen

$$V_T = (W_{MCPA} \times 0,615 + W_{MCPM} \times 0,5 + W_{OPA} \times 1,102 + W_{SA} \times 1,101)ml/g$$

der ersten Komponente.

**24.** Zement nach einem der Ansprüche 1 bis 23, **dadurch gekennzeichnet, daß** das Verhältnis des Volumens $V_L$ der zweiten Komponente und von $V_T$ der ersten Komponente im Bereich von $0,5\ V_T \leq V_L \leq 10,0\ V_T$, vorzugsweise im Bereich von $1,2\ V_T \leq V_L \leq 2,0\ V_T$ liegt.

**25.** Zement nach einem der Ansprüche 1 bis 24, **dadurch gekennzeichnet, daß** die erste Komponente zudem pharmazeutisch oder physiologisch aktive Substanzen enthalten kann, vorzugsweise gewählt aus der Gruppe der Antibiotika, entzündungshemmenden Mitteln, krebswirksamen Mitteln und Knochenwachstumsfaktoren.

**26.** Zement nach Anspruch 25, **dadurch gekennzeichnet, daß** die besagten Antibiotika gewählt werden aus der Gruppe der:
Aminoglycoside, Vancomycine, Gentamicine oder Salze davon, vorzugsweise Gentamicinsulfat oder Gentamicin-Crobefat.

**27.** Zement nach Anspruch 26, **dadurch gekennzeichnet, daß** die besagten Antibiotika eine Mixtur aus Aminoglycosiden sind, vorzugsweise eine Mixtur aus Vancomycin mit Gentamicin.

**28.** Zement nach einem der Ansprüche 1 bis 27, **dadurch gekennzeichnet, daß** die erste Komponente mindesten 50 Gewichtsprozent beta-tertiäres Calciumphosphat $\beta$-Ca$_3$ (PO$_4$)$_2$ ($\beta$-TCP) enthält.

**29.** Zement nach einem der Ansprüche 1 bis 28, **dadurch gekennzeichnet, daß** die Partikel der besagten dritten Komponente aus einem anderen Material als beta-tertiärem Calciumphosphat $\beta$-Ca$_3$ (PO$_4$)$_2$ ($\beta$-TCP) gemacht sind.

**30.** Zement nach Anspruch 29, **dadurch gekennzeichnet, daß** die Partikel der besagten dritten Komponente aus einem Material gemacht sind, gewählt aus der Gruppe der:

   Hydroxyapatite;
   biphasische Calciumphosphatgemische (HA/$\beta$-TCP);
   Biogläser;
   oder polymerische Materialien.

**31.** Zement nach einem der Ansprüche 1 bis 30, **dadurch gekennzeichnet, daß** die dritte Komponente 1 bis 99 Volumenprozent der besagten gehärteten Masse bildet.

**32.** Zement nach einem der Ansprüche 1 bis 31, **dadurch gekennzeichnet, daß** die dritte Komponente ein Calciumphosphat mit einem Ca/P-Verhältnis unter 1,5, und vorzugsweise im Bereich von 1,35 bis 1,49 ist.

33. Zement nach einem der Ansprüche 1 bis 31, **dadurch gekennzeichnet, daß** die dritte Komponente ein Calciumphosphat mit einem Ca/P-Verhältnis über 1,5, und vorzugsweise Hydroxyapatit ist.

34. Methode für die Fertigung einer Brushitmatrix $CaHPO_4$. $2H_2O$ (DCPD) als einstweiliges Knochenersatzmaterial, in dem die besagten drei Komponenten nach einem der Ansprüche 1 bis 33 vermischt werden, um ihre Erhärtung zu ermöglichen.

35. Vorübergehendes Knochenersatzmaterial nach Anspruch 34, erhalten durch eine Methode nach Anspruch 34, **dadurch gekennzeichnet, daß** es Brushit $CaHPO_4$. $2H_2O$ (DCPD) enthält.

36. Vorübergehendes Knochenersatzmaterial nach Anspruch 35, **dadurch gekennzeichnet, daß** es beta-tertiäre Cal- ciumphosphatpartikel $\beta$-$Ca_3$ $(PO_4)_2$ ($\beta$-TCP) enthält, die in die besagte Brushitmatrix eingebettet sind.


**Revendications**

1. Ciment pour applications chirurgicales comprenant :

    un premier composant comprenant :

        des particules de bêta-phosphate de calcium tertiaire $\beta$-$Ca_3(PO_4)_2$ ($\beta$-TCP); et
        des particules de phosphate monocalcique $Ca(H_2PO_4)_2$ (MCPA) ou de monohydrate de phosphate mo- nocalcique $Ca(H_2PO_4)_2.H_2O$ (MCPM) ou de l'acide phosphorique;

    un second composant comprenant de l'eau; et
    un troisième composant comprenant des particules d'un diamètre moyen qui est plus grand que le diamètre moyen des particules de bêta-phosphate de calcium tertiaire $\beta$-$Ca_3(PO_4)_2$ ($\beta$-TCP) précitées du premier composant,

    de telle sorte qu'après le mélange des trois composants précités se forme une masse durcie comprenant de la brushite $CaHPO_4.2H_2O$ (DCPD),
    **caractérisé en ce que** :

        a) les particules de $\beta$-TCP ont une aire superficielle spécifique moyenne inférieure à 10.000 $m^2$/g;
        b) le troisième composant est un phosphate de calcium avec un rapport Ca/P différent de 1,5;
        c) le troisième composant constitue 1 à 99 % en volume de la masse durcie précitée; et
        d) le premier composant comprend de plus un agent de contrôle de vitesse de prise.

2. Ciment suivant la revendication 1, **caractérisé en ce que** le premier composant comprend des particules de bêta- phosphate de calcium tertiaire $\beta$-$Ca_3(PO_4)_2$ ($\beta$-TCP) et des particules de phosphate monocalcique $Ca(H_2PO_4)_2$ (MCPA) ou de monohydrate de phosphate monocalcique $Ca(H_2PO_4)_2.H_2O$ (MCPM).

3. Ciment suivant l'une ou l'autre des revendications 1 et 2, **caractérisé en ce que** le temps de prise dudit ciment tel que mesuré à 25°C est d'au moins 2 minutes.

4. Ciment suivant la revendication 3, **caractérisé en ce que** le temps de prise dudit ciment tel que mesuré à 25°C est d'au moins 3 minutes.

5. Ciment suivant la revendication 4, **caractérisé en ce que** le temps de prise dudit ciment tel que mesuré à 25°C est d'au moins 5 minutes.

6. Ciment suivant l'une quelconque des revendications 1 à 5, **caractérisé en ce que** les particules de $\beta$-TCP ont une aire superficielle spécifique moyenne de 0,0137-2,000 $m^2$/g.

7. Ciment suivant la revendication 6, **caractérisé en ce que** les particules de $\beta$-TCP ont une aire superficielle spécifique moyenne de 0,8-1,5 $m^2$/g.

**8.** Ciment suivant la revendication 7, **caractérisé en ce que** le premier composant est divisé en trois sous-composants X, Y et Z, le sous-composant X comprenant les particules de MCPA et/ou de MCPM, le sous-composant Y comprenant les particules de β-TCP et l'agent de contrôle de vitesse de prise et le sous-composant Z comprend le troisième composant.

**9.** Ciment suivant l'une ou l'autre des revendications 7 et 8, **caractérisé en ce que** l'agent de contrôle de vitesse de prise est choisi dans le groupe comprenant le pyrophosphate de sodium, le pyrophosphate de potassium, l'acétate de sodium, l'acétate de potassium, le citrate de sodium, le citrate de potassium, le phosphocitrate de sodium, le phosphocitrate de potassium, le sulfate de sodium et le sulfate de potassium, l'hémihydrate de sulfate de calcium $CaSO_4.0,5\ H_2O$ (CSH), le pyrophosphate de sodium $Na_4P_2O_7.10\ H_2O$ (NaPPH), le dihydrogénopyrophosphate de sodium $Na_2H_2P_2O_7$ (NaHPP), le pyrophosphate de calcium $Ca_4P_2O_7$ (CaPP), le sulfate de magnésium et les biphosphonates de sodium et de potassium.

**10.** Ciment suivant l'une quelconque des revendications 1 à 9, **caractérisé en ce que** le second composant comprend de plus de l'acide orthophosphorique (OPA) et/ou de l'acide sulfurique (SA).

**11.** Ciment suivant l'une quelconque des revendications 1 à 10, **caractérisé en ce que** le poids total $W_{TCP}$ des particules de β-TCP du premier et du troisième composant est plus grand que le poids

$$W_T = W_{MCPA}/0,7546 + W_{MCPM}/0,8127 + W_{OPA}/0,3159 + W_{SA}/0,3162$$

où $W_{MCPA}$, $W_{MCPM}$, $W_{OPA}$ et $W_{SA}$ sont, respectivement, les poids de MCPA, MCPM, OPA et SA utilisés.

**12.** Ciment suivant la revendication 11, **caractérisé en ce que** le poids $W_{TCP}$ se situe dans la gamme de $1,2\ W_T \leq W_{TCP} \leq 10,0\ W_T$.

**13.** Ciment suivant la revendication 12, **caractérisé en ce que** le poids $W_{TCP}$ se situe dans la gamme de $2\ W_T \leq W_{TCP} \leq 5\ W_T$.

**14.** Ciment suivant l'une quelconque des revendications 1 à 13, **caractérisé en ce que** le diamètre de particule moyen du troisième composant est au moins deux fois, avantageusement au moins 10 fois plus grand que le diamètre moyen des particules de bêta-phosphate de calcium tertiaire β-$Ca_3(PO_4)_2$ (β-TCP) précité du premier composant.

**15.** Ciment suivant l'une quelconque des revendications 1 à 14, **caractérisé en ce que** le diamètre de particule moyen du troisième composant se situe dans la gamme de 50 à 2.000 μm, avantageusement entre 250 et 750 μm.

**16.** Ciment suivant l'une quelconque des revendications 1 à 15, **caractérisé en ce que** le troisième composant comprend des particules d'hydroxyapatite.

**17.** Ciment suivant l'une quelconque des revendications 1 à 15, **caractérisé en ce que** le troisième composant comprend des particules polymériques.

**18.** Ciment suivant l'une quelconque des revendications 1 à 17, **caractérisé en ce que** deux types différents de particules de β-TCP sont utilisés, le premier type étant constitué de particules d'une taille de particule moyenne de 5 μm avec moins de 10% en volume des particules plus petites que 1 μm, et le second type étant constitué de particules d'un diamètre moyen allant de 150 à 500 μm, avantageusement allant de 250 à 400 μm.

**19.** Ciment suivant l'une quelconque des revendications 1 à 18, **caractérisé en ce que** le rapport atomique Ca/P des particules de β-TCP du premier composant est compris entre 1,350 et 1,499, avantageusement entre 1,45 et 1,47.

**20.** Ciment suivant la revendication 19, **caractérisé en ce que** le rapport atomique Ca/P précité est compris entre 1,455 et 1,465.

**21.** Ciment suivant l'une quelconque des revendications 1 à 20, **caractérisé en ce que** l'un des deux composants comprend de plus un polymère biodégradable.

**22.** Ciment suivant la revendication 21, **caractérisé en ce que** le polymère biodégradable est choisi dans le groupe comprenant les dérivés de polysaccharides, avantageusement l'acide hyaluronique, le dextrane, l'hydroxypropylméthyl cellulose, les dérivés de chitine, avantageusement le chitosane, la gomme de xanthane, l'agarose, les polyéthylèneglycols (PEG), les méthacrylates de polyhydroxyéthylène (HEMA), les protéines synthétiques et naturelles et les collagènes.

**23.** Ciment suivant l'une quelconque des revendications 1 à 22, **caractérisé en ce que** le volume $V_L$ du second composant est égal ou supérieur au volume

$$V_T = (W_{MCPA} \times 0,615 + W_{MCPM} \times 0,5 + W_{OPA} \times 1,102 + W_{SA} \times 1,101) \text{ ml/g}$$

du premier composant.

**24.** Ciment suivant l'une quelconque des revendications 1 à 23, **caractérisé en ce que** la relation de volume $V_L$ du second composant et de $V_T$ du premier composant se situe dans la gamme de $0,5 \ V_T \leq V_L \leq 10,0 \ V_T$, avantageusement dans la gamme de $1,2 \ V_T \leq V_L \leq 2,0 \ V_T$.

**25.** Ciment suivant l'une quelconque des revendications 1 à 24, **caractérisé en ce que** le premier composant comprend de plus des substances pharmaceutiquement ou physiologiquement actives, avantageusement choisies dans le groupe des antibiotiques, des anti-inflammatoires, des médicaments anticancéreux et des facteurs de croissance osseuse.

**26.** Ciment suivant la revendication 25, **caractérisé en ce que** lesdits antibiotiques sont choisis dans le groupe comprenant les aminoglycosides, les vancomycines, les gentamycines et leurs sels, avantageusement le sulfate de gentamycine ou la matière grasse "crobefat" de gentamycine.

**27.** Ciment suivant la revendication 26, **caractérisé en ce que** l'antibiotique est un mélange d'aminoglycosides, avantageusement un mélange de vancomycine et de gentamycine.

**28.** Ciment suivant l'une quelconque des revendications 1 à 27, **caractérisé en ce que** le premier composant comprend au moins 50 % en poids de bêta-phosphate de calcium tertiaire $\beta$-$Ca_3(PO_4)_2$ ($\beta$-TCP)

**29.** Ciment suivant l'une quelconque des revendications 1 à 28, **caractérisé en ce que** les particules du troisième composant sont faites d'une autre matière que le bêta-phosphate de calcium tertiaire $\beta$-$Ca_3(PO_4)_2$ ($\beta$-TCP).

**30.** Ciment suivant la revendication 29, **caractérisé en ce que** les particules du troisième composant sont faites d'une matière choisie dans le groupe comprenant l'hydroxyapatite, les mélanges à base de phosphate de calcium biphasique (HA/$\beta$-TCP), les bioverres et les matières polymériques.

**31.** Ciment suivant l'une quelconque des revendications 1 à 30, **caractérisé en ce que** le troisième composant constitue 1 à 99 % en volume de la masse durcie précitée.

**32.** Ciment suivant l'une quelconque des revendications 1 à 31, **caractérisé en ce que** le troisième composant est un phosphate de calcium avec un rapport Ca/P inférieur à 1,5, avantageusement allant de 1,35 à 1,49.

**33.** Ciment suivant l'une quelconque des revendications 1 à 31, **caractérisé en ce que** le troisième composant est un phosphate de calcium avec un rapport Ca/P supérieur à 1,5, avantageusement de l'hydroxyapatite.

**34.** Procédé de production d'une matrice de brushite $CaHPO_4.2H_2O$ (DCPD) comme matière de remplacement osseuse temporaire, **caractérisé en ce que** les trois composants précités suivant l'une quelconque des revendications 1 à 33 sont mélangés ensemble et laissés durcir.

**35.** Matière de remplacement osseuse temporaire obtenue par le procédé suivant la revendication 34, **caractérisé en ce qu'**elle comprend de la brushite $CaHPO_4.2H_2O$ (DCPD).

**36.** Matière de remplacement osseuse temporaire suivant la revendication 35, **caractérisée en ce qu'**elle comprend des particules de bêta-phosphate de calcium tertiaire $\beta$-$Ca_3(PO_4)_2$ ($\beta$-TCP) incorporées dans la matrice de brushite précitée.